(19) Europäisches Patentamt
European Patent Office
Office européen des brevets

(11) **EP 4 744 768 A1**

(12) **EUROPEAN PATENT APPLICATION**
published in accordance with Art. 153(4) EPC

(43) Date of publication:
**20.05.2026 Bulletin 2026/21**

(21) Application number: **24856576.4**

(22) Date of filing: **11.04.2024**

(51) International Patent Classification (IPC):
**B01J 19/26** (2006.01)   **B01J 19/24** (2006.01)
**B01J 4/00** (2006.01)   **C07C 17/10** (2006.01)
**C07C 21/067** (2006.01)

(52) Cooperative Patent Classification (CPC):
**B01J 4/00; B01J 19/24; B01J 19/26; C07C 17/10; C07C 21/067**

(86) International application number:
**PCT/KR2024/004806**

(87) International publication number:
**WO 2025/041956 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 KR 20230108334**

(71) Applicant: Hanwha Solutions Corporation
**Jung-gu**
**Seoul 04541 (KR)**

(72) Inventors:
• KIM, Joon Hwan
  **Daejeon 34128 (KR)**
• JANG, Namjin
  **Daejeon 34128 (KR)**
• HAN, Keedo
  **Daejeon 34128 (KR)**

(74) Representative: **Berggren Oy**
**P.O. Box 16**
**Fabianinkatu 21**
**00101 Helsinki (FI)**

(54) **REACTOR FOR PREPARING ALLYL CHLORIDE**

(57)    The present disclosure relates to a reactor for preparing allyl chloride. More particularly, the present disclosure relates to a reactor for preparing allyl chloride capable of improving dispersibility of raw materials and inducing a uniform reaction inside the reactor, thereby improving selectivity and yield of allyl chloride.

【FIG. 6】

EP 4 744 768 A1

**Description**

[TECHNICAL FIELD]

Cross-Reference to Related Applications

This application claims the benefit of priority based on Korean Patent Applications No. 10-2023-0108334 filed on August 18, 2023, and all disclosures in the document of the said Korean Patent Application are comprised as a part of this specification.

The present disclosure relates to a reactor for preparing allyl chloride. More particularly, the present disclosure relates to a reactor for preparing allyl chloride capable of improving dispersibility of raw materials and inducing a uniform reaction inside the reactor, thereby improving selectivity and yield of allyl chloride.

[BACKGROUND OF ART]

Generally, allyl chloride (ALC) is produced by reacting propylene ($C_3H_6$) and chlorine ($Cl_2$) in a gas phase at a high temperature. Typically, the gas-phase reaction of propylene and chlorine starts to react in the gas phase at 300°C, and impurities are generated.

Therefore, a mixing method of propylene and chlorine is very important in order to suppress the generation of impurities and increase the yield of allyl chloride in the gas-phase reaction process.

Generation ratios of allyl chloride and impurities vary significantly depending on a temperature distribution formed inside the reactor. Therefore, it is required to appropriately maintain the temperature distribution during a gas-phase reaction process inside the reactor.

In particular, in order to suppress generation of 1,2-DCPa (1,2-dichloropropane), which is mainly generated in a low-temperature region, it is necessary to increase a temperature of reactants injected into the reactor. However, on the other hand, injecting high-temperature reactants has a problem in that coke is generated.

Therefore, in order to suppress the generation of coke inside the reactor, a back-mixing method is used in which heat generated by an exothermic reaction inside the reactor is supplied to the injected reactants to increase the temperature of the reactants.

However, in a conventional can-shaped reactor installed horizontally on a floor surface, a uniform internal dynamic profile is not formed in upper and lower regions of the reactor during a back-mixing process, and thus vibration and noise may be generated.

In addition, since vortices are generated at an inlet portion of the reactor, smooth flow of fluid is not formed inside a reaction space of the reactor, and thus problems may occur in that the inlet portion of the reactor is blocked by coke, resulting in a decrease in yield of allyl chloride.

In order to improve this, Korean Unexamined Patent Publication No. 2019-0064979 proposed an allyl chloride reactor including a reaction body bilaterally symmetrically formed in a round shape with respect to a center line extending downward from a position of an inlet, and a reactant injector which is installed inside the reaction body through the inlet and in which a plurality of injection nozzles for injecting reactants into the reaction space are radially symmetrically arranged.

However, in the reactor disclosed in the above publication, dispersibility of propylene and chlorine is insufficient, and thus the reactants are not sufficiently mixed over an entire region of the reactor, resulting in a low conversion rate of propylene. In addition, in a reaction region in which a temperature is not sufficiently high, allyl chloride additionally reacts, and thus generation of by-products increases.

[DETAILED DESCRIPTION OF THE INVENTION]

[Technical Problem]

In order to solve the above-described problems, the present disclosure provides a reactor for preparing allyl chloride capable of improving dispersibility of raw materials and inducing a uniform reaction inside the reactor, thereby improving selectivity and yield of allyl chloride and reducing generation of by-products.

[Technical Solution]

The reactor for preparing allyl chloride according to one embodiment of the present disclosure includes

a reaction body in which a reaction space is formed, wherein an inlet communicating with the reaction space is formed at an upper side thereof, and the reaction body is formed in a round shape bilaterally symmetrical with respect to a center line connecting downward from a position of the inlet; and

a reactant injector which is installed inside the reaction body through the inlet and in which a plurality of injection nozzles for injecting reactants into the reaction space are radially symmetrically arranged,

wherein the reactant injector includes

a first injection body in which a first injection flow path through which propylene flows is formed, and a second injection body which is formed inside the first injection body and in which a second injection flow path through which chlorine flows is formed; and

first injection nozzles formed at an end of the first injection body and through which the propylene is injected into the reaction space, and second injection nozzles formed at an end of the second injection body and through which the chlorine is injected into the reaction space,

and satisfies the following Equation 1:

$$12\% \leq (\text{sum of cross-sectional areas of first injection nozzles / cross-sectional area of first injection body}) \times 100 \leq 18\% \qquad \text{[Equation 1]}$$

The reaction body may include a first reaction part which is formed with a first curvature downward from a position at which the inlet is formed and forms a first portion of the reaction body; and

a second reaction part which is connected to the first reaction part and is formed with a second curvature different from the first curvature and forms a second portion of the reaction body.

The first reaction part may be formed with an elliptical first curvature.

The second reaction part may be formed with a hemispherical second curvature.

The first curvature and the second curvature may be formed at a curvature ratio of 1:1 to 5:1, specifically 1.5:1 to 4:1, more specifically 1.7:1 to 2.3:1, and even more specifically 1.9:1 to 2.1:1

The first injection nozzles and the second injection nozzles may be formed on a cone portion protruding from an end of the injection body.

The first injection nozzles and the second injection nozzles may be connected in a bent state from the first injection flow path and the second injection flow path, and radially inject the reactants to an outside of the cone portion.

The first injection nozzles and the second injection nozzles may each be provided in a number from 2 to 10.

[ADVANTAGEOUS EFFECTS]

According to one embodiment of the present disclosure, a ratio of cross-sectional areas of injection nozzles for injecting propylene into the reactor relative to a cross-sectional area of a propylene injection body satisfies a predetermined range.

Accordingly, dispersibility of raw materials inside the reactor is improved and fluidity inside the reactor is enhanced, so that by-products generated by an additional reaction of allyl chloride with chlorine inside the reactor can be suppressed, and selectivity and yield of allyl chloride can be improved.

[BRIEF DESCRIPTION OF THE DRAWINGS]

FIG. 1 is a perspective view schematically illustrating a reactor for preparing allyl chloride according to one embodiment of the present disclosure.

FIG. 2 is a side view schematically illustrating the reactor for preparing allyl chloride of FIG. 1.

FIG. 3 is a side cross-sectional view schematically illustrating the reactor for preparing allyl chloride of FIG. 1.

FIG. 4 is a cross-sectional view schematically illustrating a state in which reactants are injected from injection nozzles inside the reactor for preparing allyl chloride according to one embodiment of the present disclosure.

FIG. 5 is a partial cross-sectional view schematically illustrating a state in which reactants of propylene and chlorine are injected from injection nozzles according to one embodiment of the present disclosure.

FIG. 6 is a cross-sectional view schematically illustrating a cross section of a reactant injector in which injection nozzles are formed according to one embodiment of the present disclosure.

[DETAILED DESCRIPTION OF THE EMBODIMENTS]

Hereinafter, embodiments of the present invention will be described in detail with reference to the accompanying drawings so that those skilled in the art can readily practice the present invention. However, the present invention may be embodied in various different forms and is not limited to the embodiments described herein. In the drawings, portions irrelevant to the description are omitted for clarity, and throughout the specification, the same or similar reference numerals designate the same or similar components.

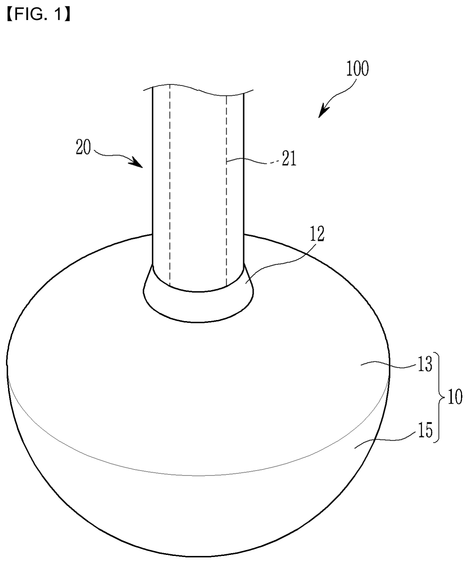

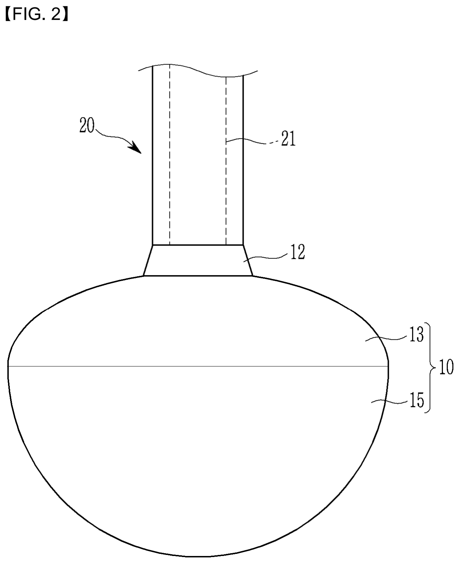

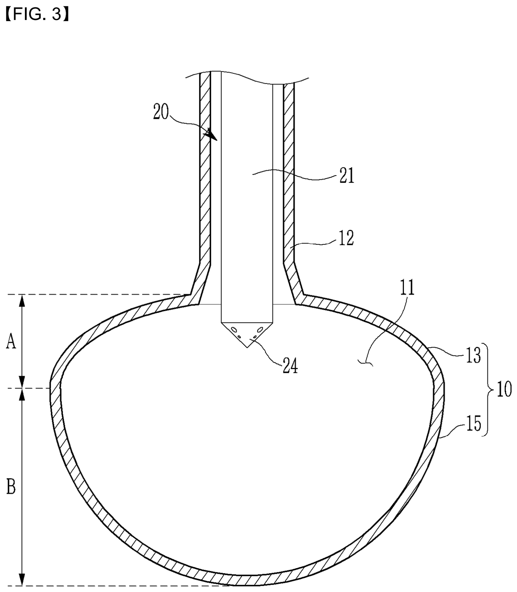

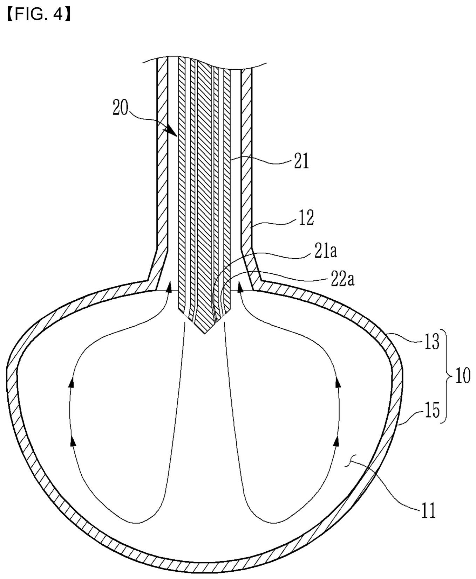

FIG. 1 is a perspective view schematically illustrating a reactor for preparing allyl chloride according to one embodiment of the present disclosure, FIG. 2 is a side view schematically illustrating the reactor for preparing allyl chloride of FIG. 1, FIG. 3 is a side cross-sectional view schematically illustrating the reactor for preparing allyl chloride of FIG. 1, and FIG. 4 is a cross-sectional view schematically illustrating a state in which reactants are injected from injection nozzles inside the reactor for preparing allyl chloride according to one embodiment of the present disclosure.

As illustrated in FIGS. 1 to 4, a reactor for preparing allyl chloride (100) according to one embodiment of the present disclosure includes a reaction body (10) in which a reaction space (11) is formed, in which an inlet (12) communicating with the reaction space (11) is formed at an upper side thereof, and which is formed in a round shape bilaterally symmetrical with respect to a center line extending downward from a position of the inlet (12), and a reactant injector (20) which is installed inside the reaction body (10) through the inlet (12) and in which a plurality of first and second injection nozzles (21a, 22a) for injecting reactants into the reaction space (11) are radially symmetrically formed.

The reactor for preparing allyl chloride (100) described below is for producing allyl chloride by gas-phase reacting reactants of propylene and chlorine at a high temperature and using a reaction (back-flow) that utilizes the temperature of the reactants. This will be described in detail below.

The reaction body (10) has a reaction space (11) formed therein, and an inlet (12) for injecting reactants may protrude from an upper side thereof. The inlet (12) may protrude as one inlet at an upper central position of the reaction body (10).

The reaction body (10) may be formed in a round shape in which curvatures vary downward from an upper side at which the inlet (12) is formed.

More specifically, the reaction body (10) may include a first reaction part (13) which is formed with a first curvature downward from a position at which the inlet (12) is formed and forms a first portion (A) of the reaction body (10), and a second reaction part (15) which is connected to the first reaction part (13), is formed with a second curvature different from the first curvature, and forms a second portion (B) of the reaction body (10).

The first reaction part (13) refers to a portion in which the inlet (12) is formed at an upper side thereof and into which reactants (22) injected through the reactant injector (20) described later are primarily introduced.

In the present embodiment, the first reaction part (13) is formed with an elliptical first curvature, such that a reaction space corresponding to the first reaction part (13) may be formed in an elliptical shape.

As such, the first reaction part (13) is formed in an elliptical shape in order to produce allyl chloride under optimal conditions. This will be described in more detail below.

The second reaction part (15) is formed to be connected to a lower portion of the first reaction part (13), and is formed with a hemispherical second curvature, such that a reaction space corresponding to the second reaction part (15) may be formed in a hemispherical shape.

As such, production of allyl chloride in an optimized state inside the reaction body (10) including the first reaction part (13) and the second reaction part (15) will be described in more detail below.

In a reaction process of obtaining allyl chloride from reactants of propylene and chlorine injected into the reaction body (10), formation of 1,3-dichloropropene and 1,2-dichloropropane, which are major by-products, occurs, and each thereof may be generated through a competitive reaction and a consecutive reaction.

Here, the competitive reaction occurs near an inlet entrance of the inlet (12) of the reaction body (10), and injection of high-temperature reactants is required.

However, when high-temperature reactants are injected inside the reaction body (10), the inlet (12) may be blocked and closed due to formation of coke, or a wall surface of an outlet through which products generated in the reaction body (10) are discharged may be contaminated or blocked.

Accordingly, in order to suppress formation of coke as much as possible, in the present embodiment, reactants (22) may be injected at a low temperature inside the reaction body (10), and a reaction (back-flow) in which heat generated by an exothermic reaction inside the reaction body (10) is returned and supplied to a position of the inlet (12) to increase a temperature of the reactants (22) may be performed.

For this purpose, in the present embodiment, the first reaction part (13) constituting the reaction body (10) may be formed in an elliptical shape, and the second reaction part (15) may be formed in a hemispherical shape.

Accordingly, reaction heat generated in a state in which reactants (22) are injected inside the reaction body (10) is smoothly circulated along inner wall surfaces of the first reaction part (13) and the second reaction part (15), and supplied to the inlet (12) portion, thereby increasing a temperature of the reactants injected into the inlet (12) portion.

In addition, forming the first reaction part (13) and the second reaction part (15) constituting the reaction body (10) in elliptical and hemispherical shapes, respectively, may prevent occurrence of vibration or resonance phenomena.

That is, in the case of a conventional horizontal reaction body installed horizontally at an installation site in a can shape, when a reaction (back-flow) that utilizes a temperature of reactants is performed in order to increase a temperature of injected materials, vibration and noise may be generated.

That is, since the conventional horizontal reaction body is manufactured in a can shape installed in a horizontal direction, a reaction (back-flow) that utilizes a temperature of reactants occurs in different forms in upper and lower regions of the reaction body, such that a uniform internal profile is not formed, thereby vibration and noise may be generated.

In addition, in the conventional horizontal reaction body, collision occurs at an inlet side into which reactants are injected and vortices are generated, such that it becomes difficult to form a smooth flow direction inside the horizontal reaction body. This may cause coke to be generated at a front portion of the reaction body and cause blockage, or may break symmetry inside the reaction body, thereby making it difficult to perform production through a reaction (back-flow) that utilizes a temperature of reactants effectively.

Accordingly, the reaction body (10) of the present embodiment is implemented as a vertically symmetrical reactor formed with an elliptical first reaction part (13) at an upper side and a hemispherical second reaction part (15) at a lower side, such that reaction heat generated in the reaction space (11) of the reaction body (10) can be smoothly moved along inner wall surfaces of the reaction space (11).

That is, a reaction (back-flow) in which a temperature of reactants generated inside the reaction body (10) is supplied toward the inlet direction may be smoothly moved toward the inlet (12) direction along hemispherical and elliptical inner wall surfaces of the reaction space (11). Accordingly, an increase in temperature of low-temperature reactants discharged at the inlet (12) portion of the reaction body (10) can be effectively achieved by heat self-generated by reactants inside the reaction body (10) without additional heat supply.

Further, the reaction body (10) of the present embodiment is formed to include an elliptical first reaction part (13) and a hemispherical second reaction part (15), such that reaction action of reactants can be smoothly and effectively performed. For this purpose, a ratio between a first curvature of the first reaction body (10) and a second curvature of the second reaction body (10) may be set in a range of 1:1 to 5:1.

Accordingly, since reaction action of reactants (22) occurs smoothly and effectively inside the reaction body (10) and closure of the inlet (12) portion does not occur, it is possible to effectively prevent vibration noise such as resonance generated when reactants collide at a closed inlet portion.

When a ratio between the first curvature and the second curvature of the reaction body (10) of the present embodiment is in a range of 1:1 to 5:1, selectivity of allyl chloride produced inside the reaction body may be improved.

Meanwhile, reactants may be injected inside the reaction body (10) by a reactant injector (20).

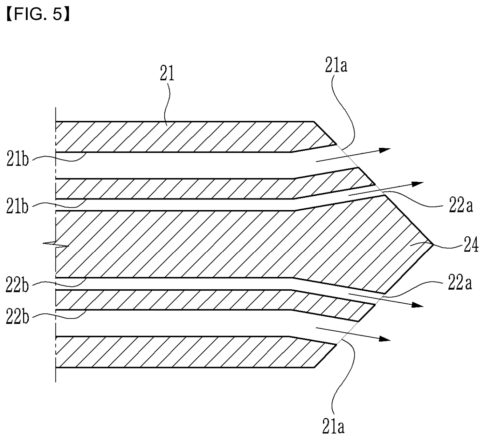

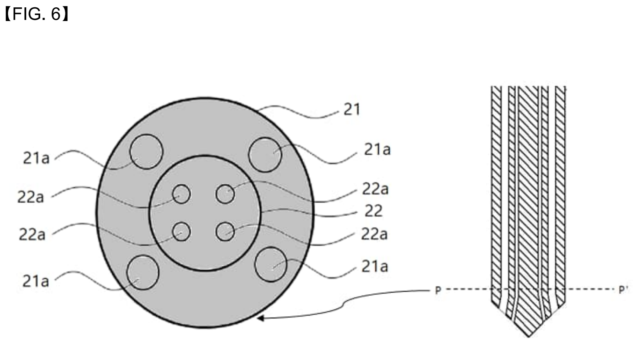

FIG. 5 is a partial cross-sectional view schematically illustrating a state in which reactants of propylene and chlorine are sprayed from injection nozzles according to an embodiment of the present disclosure, and FIG. 6 is a schematic cross-sectional view illustrating a reactant injector in which injection nozzles are formed according to an embodiment of the present disclosure.

As illustrated in FIGS. 5 and 6, the reactant injector (20) includes a first injection body (21) in which a first injection flow path (21b) through which propylene flows is formed, and a second injection body (22) which is formed inside the first injection body (21) and in which a second injection flow path (22b) through which chlorine flows is formed.

An end of the first injection body (21) includes a plurality of first injection nozzles (21a) through which propylene that has flowed through the first injection flow path (21b) is injected inside the reaction space (11). In addition, an end of the second injection body (22) includes a plurality of second injection nozzles (22a) through which chlorine that has flowed through the second injection flow path (22b) is injected inside the reaction space (11).

Although the drawings illustrate that four first injection nozzles (21a) and four second injection nozzles (22a) are provided, respectively, the present invention is not limited thereto, and the first and second injection nozzles (21a, 22a) may each be independently provided in plural numbers, that is, two or more, for example, from two to ten.

The reactant injector (20) may be inserted into the inlet (12) and installed at a position of the inlet (12) to supply propylene and chlorine inside the reaction body (10). Propylene and chlorine injected through the first injection flow path (21b) and the second injection flow path (22b) of the reactant injector (20) may be appropriately injected and supplied inside the reaction body (10) through the first injection nozzles (21a) and the second injection nozzles (22a), respectively.

The first injection nozzles (21a) and the second injection nozzles (22a) are formed at an end of the reactant injector (20). In the present embodiment, propylene and chlorine are illustratively described as being injected through four nozzles, respectively, but the present invention is not limited thereto. Propylene and chlorine may be radially injected inside the reaction body (10) in a state of being divided at the respective first injection nozzles (21a) and second injection nozzles (22a).

As such, the first injection nozzles (21a) and the second injection nozzles (22a) are formed radially in order to increase a contact area with heat generated and transferred by a back-flow reaction inside the reaction body (10), thereby allowing a temperature-increasing operation of reactants to be smoothly performed at a position of the inlet (12).

Here, in order to further enhance a spraying effect, the first injection nozzles (21a) and the second injection nozzles (22a) may be formed on a cone portion (24).

That is, the cone portion (24) may protrude in an inclined manner from an end of the reactant injector (20). A plurality of first injection nozzles (21a) and second injection nozzles (22a) are radially formed on inclined side surfaces of the cone portion (24), such that the first injection nozzles (21a) and the second injection nozzles (22a) may be connected to the first injection flow path (21b) and the second injection flow path (22b) of the reactant injector (20) in a bent state.

Accordingly, propylene and chlorine are injected inside the reaction body (10) by the first injection nozzles (21a) and the second injection nozzles (22a) formed on the side surfaces of the cone portion (24) with a wider spraying area, such that an area of contact with heat transferred by a back-flow reaction can be more effectively increased.

In the present embodiment, the first injection nozzles (21a) and the first injection body (21) satisfy Equation 1 below:

$$12\% \leq (\text{sum of cross-sectional areas of first injection nozzles / cross-sectional area of first injection body}) \times 100 \leq 18\% \qquad \text{[Equation 1]}$$

According to Equation 1, a ratio of the sum of the cross-sectional areas of the first injection nozzles to the cross-sectional area of the first injection body may be 12% or more, 13% or more, or 14% or more, and 18% or less, 17% or less, 16% or less, or 15% or less.

When the ratio of the sum of the cross-sectional areas of the first injection nozzles to the cross-sectional area of the first injection body is 12% or more and 18% or less, selectivity of allyl chloride may be improved, and a generation ratio of by-products, in particular, 1,2-DCPa, may be significantly reduced.

Referring to FIG. 6, the cross-sectional area of the first injection body (21) in Equation 1 is an area obtained by cutting an end of the reactant injector (20) along a line P-P' perpendicular to a longitudinal direction, and corresponds to a total area surrounded by an outer circumferential surface of the first injection body (21), that is, a gray region shown in FIG. 6.

In addition, the sum of the cross-sectional areas of the first injection nozzles refers to a total sum of the cross-sectional areas of the plurality of first injection nozzles (21a).

Here, the line P-P' is defined as an imaginary line that is perpendicular to a longitudinal direction of the reactant injector (20) at a cylindrical portion of the reactant injector (20), excluding a cone portion protruding in an inclined conical shape.

A temperature distribution inside the reactor varies depending on a flow pattern of raw materials, and this is related to productivity of allyl chloride. In addition, fluidity of raw materials inside the reactor is related to dispersibility of raw materials injected from nozzles. In particular, it has been recognized that a nozzle flow velocity is controlled according to a ratio of the cross-sectional areas of the first injection nozzles to the cross-sectional area of the first injection body into which propylene is injected, and that this control improves dispersibility of raw materials and fluidity inside the reactor. Accordingly, when dispersibility at the first injection nozzles is improved, by-products generated by an additional reaction of allyl chloride with chlorine in a region where a temperature is not sufficiently high can be suppressed, and selectivity and yield of allyl chloride can be improved.

Meanwhile, Table 1 below shows selectivity of allyl chloride, selectivity of a by-product DCPa, and a generation ratio of 1,2-DCPa, when a ratio of the sum of the cross-sectional areas of the first injection nozzles (21a) relative to the cross-sectional area of the first injection body (21) in Equation 1 is 12%, 15%, or 20%.

[Table 1]

|  | Example 1 | Example 2 | Comparative Example 1 |
|---|---|---|---|
| Equation 1 | 12% | 15% | 20% |
| Allyl chloride selectivity [%] | 78.3 | 76.3 | 75.3 |
| 1,2-DCPa selectivity [%] | 8.6 | 12 | 13.5 |
| Generation ratio of 1,2-DCPa * | 0.62 | 0.88 | 1 |
| (*The generation ratio of 1,2-DCPa refers to a relative generation ratio when a generation amount of 1,2-DCPa in Comparative Example 1 is set to 1.) | | | |

Referring to Table 1, it can be confirmed that, when the cross-sectional area ratio of Equation 1 satisfies a predetermined range, selectivity of allyl chloride is improved and generation of by-products is significantly reduced.

Although preferred embodiments of the present invention have been described above, the present invention is not limited thereto, and various modifications may be made within the scope of the claims, the detailed description, and the accompanying drawings, and such modifications also fall within the scope of the present invention.

[DESCRIPTION OF SYMBOLS]

| 10 | reaction body | 11 | reaction space |
|----|---------------|----|----------------|
| 12 | inlet | 13 | first reaction part |
| 15 | second reaction part | 20 | reactant injector |
| 21 | first injection body | 22 | second injection body |
| 21a | first injection nozzle | 22a | second injection nozzle |
| 24 | cone portion | | |

**Claims**

1. A reactor for preparing allyl chloride, comprising

   a reaction body in which a reaction space is formed, wherein an inlet communicating with the reaction space is formed at an upper side thereof, and the reaction body is formed in a round shape bilaterally symmetrical with respect to a center line connecting downward from a position of the inlet; and
   a reactant injector which is installed inside the reaction body through the inlet and in which a plurality of injection nozzles for injecting reactants into the reaction space are radially symmetrically arranged,
   wherein the reactant injector comprises
   a first injection body in which a first injection flow path through which propylene flows is formed, and a second injection body which is formed inside the first injection body and in which a second injection flow path through which chlorine flows is formed; and
   first injection nozzles formed at an end of the first injection body and through which the propylene is injected into the reaction space, and second injection nozzles formed at an end of the second injection body and through which the chlorine is injected into the reaction space,
   and satisfies the following Equation 1:

   $12\% \leq$ (sum of cross-sectional areas of first injection nozzles / cross-sectional area of first injection body) $\times\ 100 \leq 18\%$. [Equation 1]

2. The reactor for preparing allyl chloride of Claim 1,
   wherein the reaction body comprises:

   a first reaction part which is formed with a first curvature downward from a position at which the inlet is formed and forms a first portion of the reaction body; and
   a second reaction part which is connected to the first reaction part and is formed with a second curvature different from the first curvature and forms a second portion of the reaction body.

3. The reactor for preparing allyl chloride of Claim 2,
   wherein the first reaction part is formed with an elliptical first curvature.

4. The reactor for preparing allyl chloride of Claim 3,
   wherein the second reaction part is formed with a hemispherical second curvature.

5. The reactor for preparing allyl chloride of Claim 4,
   wherein the first curvature and the second curvature are formed at a curvature ratio of 1:1 to 5:1.

6. The reactor for preparing allyl chloride of Claim 1,
   wherein the first injection nozzles and the second injection nozzles are formed on a cone portion protruding from an end of the injection body.

7. The reactor for preparing allyl chloride of Claim 6,
   wherein the first injection nozzles and the second injection nozzles are connected in a bent state from the first injection flow path and the second injection flow path, and radially inject the reactants to an outside of the cone portion.

8. 9. The reactor for preparing allyl chloride of Claim 1,
   wherein the first injection nozzles and the second injection nozzles are each provided in a number from 2 to 10.

【FIG. 1】

【FIG. 2】

【FIG. 3】

【FIG. 4】

【FIG. 5】

【FIG. 6】

## INTERNATIONAL SEARCH REPORT

| International application No. |
|---|
| **PCT/KR2024/004806** |

| A. | CLASSIFICATION OF SUBJECT MATTER |
|---|---|

**B01J 19/26**(2006.01)i; **B01J 19/24**(2006.01)i; **B01J 4/00**(2006.01)i; **C07C 17/10**(2006.01)i; **C07C 21/067**(2006.01)i

According to International Patent Classification (IPC) or to both national classification and IPC

| B. | FIELDS SEARCHED |
|---|---|

Minimum documentation searched (classification system followed by classification symbols)

B01J 19/26(2006.01); B01J 19/24(2006.01); B01J 38/04(2006.01); B01J 38/14(2006.01); C07C 17/02(2006.01); C07C 17/10(2006.01); C07C 21/067(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 염화 알릴(allyl chloride), 프로필렌(propylene), 반응기(reactor), 노즐(nozzle)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2019-0064979 A (HANWHA CHEMICAL CORPORATION) 11 June 2019 (2019-06-11) See paragraphs [0013]-[0064]; and figures 6 and 7. | 1-8 |
| A | KR 10-1999-0021869 A (THE DOW CHEMICAL COMPANY) 25 March 1999 (1999-03-25) See entire document. | 1-8 |
| A | JP 06-135868 A (TOKUYAMA CO., LTD. et al.) 17 May 1994 (1994-05-17) See entire document. | 1-8 |
| A | JP 2013-100248 A (SUMITOMO CHEMICAL CO., LTD.) 23 May 2013 (2013-05-23) See entire document. | 1-8 |
| A | KR 10-2017-0091405 A (KOREA INSTITUTE OF MACHINERY & MATERIALS et al.) 09 August 2017 (2017-08-09) See entire document. | 1-8 |

☐ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 July 2024** | **18 July 2024** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2024/004806** |

| Patent document cited in search report | | | Publication date (day/month/year) | Patent family member(s) | | | Publication date (day/month/year) |
| --- | --- | --- | --- | --- | --- | --- | --- |
| KR | 10-2019-0064979 | A | 11 June 2019 | None | | | |
| KR | 10-1999-0021869 | A | 25 March 1999 | CN | 1188466 | A | 22 July 1998 |
| | | | | EP | 0837841 | A1 | 29 April 1998 |
| | | | | EP | 0837841 | B1 | 13 March 2002 |
| | | | | EP | 0837841 | B2 | 03 May 2006 |
| | | | | EP | 1018366 | A2 | 12 July 2000 |
| | | | | EP | 1018366 | A3 | 26 July 2000 |
| | | | | EP | 1018366 | B1 | 04 February 2004 |
| | | | | JP | 11-505834 | A | 25 May 1999 |
| | | | | JP | 4047381 | B2 | 13 February 2008 |
| | | | | US | 5504266 | A | 02 April 1996 |
| | | | | US | 6004517 | A | 21 December 1999 |
| | | | | WO | 96-37450 | A1 | 28 November 1996 |
| JP | 06-135868 | A | 17 May 1994 | JP | 2813100 | B2 | 22 October 1998 |
| | | | | US | 5367105 | A | 22 November 1994 |
| JP | 2013-100248 | A | 23 May 2013 | None | | | |
| KR | 10-2017-0091405 | A | 09 August 2017 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230108334

- KR 20190064979